Europäisches Patentamt

(19) European Patent Office

Office européen des brevets

(11) Publication number: **0 301 790**
**A2**

## EUROPEAN PATENT APPLICATION

(21) Application number: 88306828.0

(51) Int. Cl.4: **A61B 5/00 , A61B 5/04**

(22) Date of filing: 25.07.88

(30) Priority: 24.07.87 US 77733

(43) Date of publication of application:
01.02.89 Bulletin 89/05

(84) Designated Contracting States:
AT BE CH DE ES FR GB GR IT LI LU NL SE

(71) Applicant: **THE BOARD OF TRUSTEES OF THE LELAND STANFORD JUNIOR UNIVERSITY**

**Stanford, CA 94305(US)**

(72) Inventor: **Lusted, Hugh S.**
**774 Allen Court**
**Palo Alto California 94305(US)**
Inventor: **Knapp, Benjamin R.**
**1735 Woodland Avenue, No. 10**
**East Palo Alto California 94301(US)**

(74) Representative: **Ayers, Martyn Lewis Stanley et al**
**J.A. KEMP & CO. 14 South Square Gray's Inn**
**London, WC1R 5EU(GB)**

(54) Biopotential digital controller for music and video applications.

(57) There is disclosed apparatus and methods for sensing electrical signals generated in a living body and mapping these signals to MIDI code. The apparatus senses electrical signals generated in the body through electrodes on the skin or implanted in various areas of the body. The electrical signals are then amplified, filtered and converted to digital data. This digital data is analyzed to determine characteristics of the electrical signals such as amplitude, frequency, change in amplitude or change in frequency. These characteristics are then mapped to MIDI code or other code to control the generation of musical notes or video display information. There is also disclosed a method and apparatus for determining the alpha wave or other content of brain waves by taking a fast fourier transform of the brain wave and determining the coefficient for the fourier frequency component at the frequency of desired frequencies. Further, a method of altering MIDI code generated from biopotentials is disclosed where the effect of musical notes generated by the MIDI code on the biopotentials causes changes in the MIDI code. A similar method is also disclosed wherein MIDI code received from another machine is changed based upon mapping of biopotentials from the body of a subject.

## BIOPOTENTIAL DIGITAL CONTROLLER FOR MUSIC AND VIDEO APPLICATIONS

### Background of the Invention

The invention relates to the field of computer generated music. Specifically, the invention relates to the field of sensing electrical signals generated in a human body and mapping those signals into MIDI code which controls the generation of musical notes.

Various methods of collecting biological data by sensing electrical signals generated by a living body and converting these signals to audible or visual manifestations have long been in use. These systems have clinical and psychotherapeutic applications. Examples of such systems are found in US Patent 4,136,684 to Scattergood et al., and US Patent 4,170,225 to Criglar et al. In Scattergood et al., an electromyographic biofeedback system is disclosed. A transducer is used to sense electrical signals from the body, and these signals are amplified by a differential amplifier to eliminate common mode noise. After further signal processing, and averaging in a third order averaging filter, the output signal from the averaging filter is used to control a current controlled oscillator which provides a series of audible pulses at a rate which varies linearly with the value of the input voltage detected by the transducer. The system is useful in detecting small muscle movements to indicate progress in rehabilitation of patients.

The Criglar reference teaches a portable biofeedback device for monitoring electrical signals produced by a muscle activity. A series of filters and preamplifiers eliminate noise components and unwanted frequency components such that only signals in a certain frequency range are amplified. The signals monitored are those produced by muscle activity. Both visual and audio feedback is generated for use by the person being monitored.

Another class of prior art devices senses brain wave activity and converts the brain waves or components thereof to visible or audible feedback signals. One such device is disclosed in US Patent 4,228,807 to Yagi et al. Yagi teaches a biofeedback device which senses brain waves and separately extracts the alpha wave component and the muscle component included in the brain wave. Yagi teaches generating auditory feedback which allows the user to recognize accurately the appearance of the alpha wave component in the brain wave, thereby indicating the state of relaxation of the subject. Audible sounds are also generated for the muscle component in the brain wave which allows the user to know when tension in the subject's mental state exists.

Another such system is taught by Shiga in US Patent 4,334,545. Shiga teaches a system to detect the brain wave components of the subject in low and high frequency bands. The low frequency band has a frequency range approximately the same as the alpha wave component generated by the brain, and the higher frequency range has a frequency which is considerably higher than the highest frequency of the brain alpha waves. Audible responses are generated. Indications are generated when the detected high frequency components have an amplitude less than a level indicative that the subject is not in a state of relaxation and when the detected low frequency component has an amplitude greater than a first predetermined value and less than a second predetermined value. When the low frequency amplitude is between these two predetermined values, the subject is generating alpha waves and is in a state of relaxation.

Another alpha wave sensing system is taught by Shiga in US Patent 4,354,505. In this reference, Shiga teaches a self-training biofeedback system which has a binary counter. The counter counts the intervals where alpha wave components are present which represents the relaxation period. A visual indicator connected to the counter provides the person being trained with a display of the measured time period when alpha waves are being generated so that the depth of relaxation can be visualized.

A method for generating sounds from brain waves is taught in US Patent 4,454,886 to Lee. In this system, the brain wave signal derived from an electroencephalograph is converted in real time from an analog to a digital signal and stored in a memory. Another circuit then detects points in time along this EEG signal which define segments of the signal. The signals in the memory are then read out in segments with the segment signals being repeatedly read out n number of times at a rate n times faster than the input rate to the memory. This generates a train of signals which are high frequency replicas of the signal segment. The segments are stored in a FIFO memory such that as the first segment to be stored is the first segment to be read out of memory, and the second segment to be stored in the memory is the second segment to be read out of the memory.

Biological activities manifested in electrical signals occurring in an individual, a couple, or a group of persons manifested in electrical signals are sensed and utilized in a system taught by Fehmi et al. in US Patent 4,031,883. Multiple electrical contacts are applied to the scalp or body of the individual or all the people in the group. The audible sensory feedback signals generated from the electrical signals detected by these electrical contacts simultaneously communicate to the subject or subjects ongoing fluctuations in frequency, amplitude and phase unity among two or more biological rhythms occurring within the individual or group of individuals. These auditory signals may be used to establish awareness and control over the functions of the brain which are being monitored, or as an aid to meditation, or as an aid to learning to flexibly and easily establish or dissolve various states of attention and consciousness associated with the characteristics of the signals being sensed.

None of the above-described prior art systems generate musical notes in response to the sensed signals, and none generates musical data in the MIDI interface code.

The only prior art system of which the Applicants are aware which generates musical notes in response to biological information is a system developed at Michigan State University by John F. Holland, Professor of Urology. In this system, on information and belief, a chemical analysis is made of urine samples. The chemical assay machine generates an analog output signal representing the components in the urine. Such an assay may be made by a liquid chromatography system. The analog output signal is coupled to a computer via an analog-to-digital converter. A musical note is assigned to each peak in the analog signal by IBM personal computer which is programmed to convert the digital stream of data to MIDI format command words. This MIDI data may be sent to a musical instrument for conversion to musical notes which are audible to the users of the system. The computer may be programmed to play a tune such as the "Star Spangled Banner" for a normal pattern of urine components.

None these prior art systems teaches a real time system which can sense electrical signals generated in the body either in muscle tissue or in the brain and convert them in real time to MIDI format command words which are then immediately converted to musical notes. Such a system would have numerous applications in such widely diverse fields as clinical analysis, biofeedback, games, musical instruments, live performances by artists, and many other applications.

## Summary of the Invention

According to the teachings of the invention, musical notes are generated in real time from electrical signals generated in a living body which are collected and processed in real time. A system is taught herein which includes transducers for sensing one or more biologically generated electrical signals present in a living body. These transducers are coupled to conversion circuitry for converting the analog electrical signals to a stream of digital data. The stream of digital data is received by a mapping unit which extracts certain information from the stream of digital data in real time and maps these characteristics into a second stream of digital data having a format capable of controlling an output device such as a music synthesizer, a video game, or some other type of device.

In the preferred embodiment, the mapping circuitry is a computer which is programmed to derive certain characteristics of the biologically generated signals (sometimes hereafter referred to as biopotentials), and map these characteristics into one or more parameters of the MIDI interface. A biopotential is an electrical signal generated by living bodies. In the preferred embodiment, the conversion means converts the amplitude of the electrical signals generated in the body to digital words which define the amplitude during a particular sample period. A computer then maps these amplitude words to corresponding note numbers in the MIDI interface code. The note numbers correspond to frequencies of notes to be generated on a music synthesizer serving as the output device. The amplitude words are then converted to "note-on" and "note-off" MIDI interface format code sequences or data packets. These MIDI data packets are then sent to the synthesizer which generates musical notes having frequencies which are mapped in response to the intensity of the electrical signals generated in the living body.

The invention is not limited to the particular mapping scheme described above for the preferred embodiment. Many different applications for the invention are possible, and each of these applications may have peculiar requirements for the function to be performed by the conversion circuitry and many different mapping functions are possible. For example, in the video area, the invention may find application in video games, computer graphics, and teaching aids. In the music area, the invention may find application in the performance arts and in professional clinical equipment for use in the offices of doctors, psychologists and psychiatrists. Other subareas within the music field which may have applications for the invention are consumer products and medical products. Consumer products which may use the invention include music toys, amateur instruments, relaxation devices, mood makers, dream manipulation devices, pet music devices, baby music devices, and group music devices. In the medical arts, the invention may find application in music therapy, orthopedic treatment and rehabilitation, paralysis rehabilitation and sleep dis order therapies or instruments for aiding sleep. In the mechanical arts, the invention may find application in radio controlled toys, prosthetics and home appliances.

Accordingly, the invention contemplates a high degree of flexibility in the particular functions performed by the conversion and computer circuitry. For example, the conversion circuitry may include several channels for gathering information in different frequency bands from signals generated by multiple transducers placed at different locations on the body. The signals in the various frequency bands may be converted to digital signals which are then transmitted to the computer. The computer may then analyze these signals for various parameters such as the frequency of the signal, the intensity of the signal, the change in the frequency of the signal or the change in the intensity. These characteristics of the signals in each channel may be mapped to any of the various parameters which may be controlled in a synthesizer or other output device via a standard interface such as MIDI. For example, if a synthesizer is used for the output device, the standard MIDI interface may be used for mapping purposes such as changes in frequency may be mapped to program number or harmonic content. The MIDI interface for synthesizers and the SMPTE interface for video devices allow control of many characteristics of the output device. These characteristics control the qualities of the output manifestation generated by the device. Any combination of mapping from the characteristics of the biologically generated signal to the parameters which may be controlled by the interface for the output device which is suitable for the particular application will be in accordance with the teachings of the invention.

Brief Description of the Drawings

Figure 1 is a block diagram of an embodiment of the invention using a music synthesizer as an output device.

Figure 2 shows a block diagram and application of the teachings of the invention to a video game application.

Figure 3 is a flow diagram of a process of collecting electrical signals from a living body and analyzing these signals for certain characteristics and generating output manifestations in real time based upon these characteristics.

Figure 4 shows a block diagram of the preferred embodiment of the invention in the form of a system which converts intensity of biological electrical signals to note frequency of musical notes generated in real time by a music synthesizer output device.

Figure 5 shows a flow chart of the preferred embodiment of the process of the invention showing a mapping function from electrical signal intensity to note frequency for real time generation of musical notes from biological electrical signals.

Figure 6 is a flow chart of the method used to map the alpha wave component of EEG signals to MIDI code.

Figure 7 is a flow diagram of the mapping process for mapping EKG signal intensity to MIDI code for a MIDI drum machine.

Figure 8 is a flow chart representing a process of altering MIDI code which was generated in response to biopotentials by reevaluating the biopotentials after the body has had a chance to react to the musical notes generated in response to the biopotentials as they were first sensed.

Figure 9 shows a flow diagram of a process for receiving MIDI code from another machine and evaluating for biopotentials from a body and then mapping the biopotentials to corresponding modifications in the incoming MIDI code and then reoutputting the modified MIDI code.

Figure 10 shows a block diagram of an embodiment of a system according to the teachings of the invention which senses four different kinds of biopotentials and receives MIDI code from another machine and for generating MIDI code in response to the biopotentials.

Detailed Description of the Preferred Embodiment

Referring to Figure 1 there is shown a block diagram of the preferred embodiment of a system according to the teachings of the invention. A human subject 10 serves as the source for electrical signals which are generated in the body of the human subject. Figure 1 shows several transducers 12, 14 and 16 attached to the head of the subject. These transducers sense brain wave signals from the human subject 10, and output signals on the signal lines 18, 20 and 22 to a preamplifier 24.

According to the teachings of the invention, the electrical signals from the human subject which are sensed by the transducers may be any type of signal including EEG brain wave signals, EKG (electrocardiogram) signals emanating from muscles of the heart or EMG signals emanating from muscles in other parts of the body, such as the eye. The positions of the transducers 12, 14 and 16 may be anywhere on the body to detect muscle movements or other electrical activity in the body. Further, although a human subject is shown, the invention is equally applicable to the bodies of animals and even to plants if electrical activity is present in a particular form of plant.

Another place where the transducer electrodes 12, 14 and 16 may be placed on the body is in the vicinity of the eye, The eyeball is essentially a D.C. dipole and generates a D.C. signal which has a polarity related to the position of the eye. When electrodes are placed on either side of the eye, this polarity may be detected. This D.C. signal becomes more negative relative to one electrode when the eye shifts in one direction and becomes more positive when the eye shifts in the other direction. In this way, eye movements shifting the eye left and right may be detected. With different placement of the electrodes about the eye, other eye movements can also be detected. Eye dipole signals may hereafter be referred to as EOG (electrooculogram) signals.

Other possible placements of the electrodes 12, 14 and 16 may detect EGG (electrogastrogram) signals.

Although only three transducers are shown in Figure 1, it is also within teachings of the invention to use multiple sets of transducers . Generally, it is preferable to use three transducers (i.e., electrodes) to sense electrical signals generated in the brain or in muscles such that common mode noise can be rejected. Having three transducers allows the use of one common electrode and two sensing electrodes. Assuming electrode 14 is the common electrode in the embodiment shown in Figure 1, common mode signals would be signals which simultaneously appear between electrodes 12 and 14 and between electrodes 16 and 14. When these common mode signals are removed, the differential mode signals are left which are the signals which are not common as between the aforementioned electrode pairs. It is these differential mode signals which represent the muscle or brain wave activity of interest.

Removal of the common mode signals and amplification of the difference mode signals is performed by the preamplifier 24. The preamplifier 24 should be a differential amplifier with a high common mode rejection ratio and low noise generation internally. Common mode rejection of 120 decibels rejection is typical but not critical. As long as a usable signal-to-noise ratio can be obtained, any performance figures of merit will suffice.

The output of the preamplifier 24 is coupled through a filtering circuit 26 to the analog input of an analog-to-digital conversion circuit 28. The purpose of the filtering circuit is to remove any 60 cycle noise which is inductively picked up by the electrodes or their connecting wires or which is present in the output of the preamplifier 24 on the signal line 30.

The analog-to-digital conversion circuit 28 samples the output of the filtering circuit 26 on line 32 at a sampling rate sufficiently high to avoid aliasing. The A/D converter 28 generates a stream of digital data on bus 34. The sampling rate of the analog-to-digital conversion circuit 28 should be at least twice as high as the fundamental component of the frequency of interest to avoid aliasing problems according to the Nyquist criteria.

In more complex embodiments where multiple filter passbands are used, separate analog-to-digital (sometimes hereafter referred to as A/D) conversion circuits for each channel may be used. Since each passband filter puts out a different frequency or band of frequencies, separate sample rates must be used on each channel to satisfy the Nyquist criteria. Those skilled in the art will appreciate how the dominant frequencies in each passband should be sampled to get an accurate digital representation of the analog signal in each passband.

In the preferred embodiment, the analog-to-digital conversion circuit 28 serves to sample the amplitude of the signal on line 32 and convert the amplitude during each sample period to a digital word representing the amplitude or intensity of the incoming analog signal. This stream of digital intensity words on bus 34 is read by a mapping computer 36.

The purpose of the mapping computer 36 is to analyze the incoming digital data on bus 34 to determine one or more characteristics of the analog signal on line 32. This characteristic is mapped to a parameter of the MIDI interface, and a MIDI format data word or words are generated by the mapping computer 36. In the preferred embodiment, the intensity of the signal on line 32 is mapped to the frequency of the musical note to be generated for that intensity word by the music synthesizer output device 38. In other embodiments, other characteristics such as the frequency of the signal on line 32 can be mapped to the note number (the note frequency) or the change in intensity or the change in frequency of the incoming analog signal may be mapped to corresponding changes in one or more parameters which may be specified in MIDI code to control the synthesizer 38.

In multi-channel embodiments, each channel may look for a different characteristic of the incoming analog signal in the same or in different passbands. Each characteristic may then have a separate mapping by the mapping computer 36 to a separate parameter in the MIDI format interface code. Examples of MIDI format parameters which may be controlled are "note on", "note off", "note number", control parameters and system exclusive messages. The complete MIDI specification is in the public domain. A form of MIDI which is used in the preferred embodiment.

MIDI is an acronym for Musical Interinstrument Digital Interface. This interface enables synthesizers, sequencers, home computers, rhythm machines and so on to be interconnected through a standard interface. There are standard MIDI interface hardward circuits and standard MIDI data formats. MIDI communication is achieved through multi-byte messages consisting of one status byte followed by one or two data bytes. There are sixteen available channels for data transmission of MIDI code words between transmitters and receivers. The contents of the MIDI interface specification are hereby incorporated by reference.

The music synthesizer 38 receives the MIDI format data words on the output bus 40 from the mapping computer and generate musical notes in accordance with the MIDI data. In the embodiment of Figure 2, the preamplifier 24 and the filtering circuit 26 perform the same functions that the corresponding circuits perform in the embodiment of Figure 1. Likewise for the digital converter 28. The mapping computer 36 in the embodiment of Figure 2 does a different mapping than the mapping computer in Figure 1, however, since the SMPTE interface format is used. In the system of Figure 2, the output device is a video game 40. This video game drives a display 42. Video game 40 may be any video game. In the application of Figure 2, the user inputs will arrive as code words which may be converted to signals which control the operation of the video game. In other words the digital data may be converted to the signals ordinarily read by the video game from the user's joystick, fire control button, etc. In some embodiments the SMPTE interface format may be used. The SMPTE interface format is well known to those skilled in the video art.

The functioning of the mapping computer 36 will be to take the stream of digital data on the bus 34 and map it according to some function data which supplies the necessary user input data to control the video game 40. The particular mapping function depends upon the particular type of input from the user that is required by the video game 40. Suitable interface circuitry for the video game 40 will take the user input from the mapping computer 36 via bus 44 and convert it to the appropriate signals to control the action of the video game 40.

In the application of Figure 2, the user may provide input to the video game 40 by thinking certain thoughts, making certain eye movements or performing certain movements of various muscles in the user's body, depending upon where the transducers 12, 14 and 16 are placed. Many suitable video games are available to serve as the output device in the application of Figure 2. Those skilled in the art will be able to easily design appropriate digital to analog conversion interface circuitry to accept the interface data on bus 44 and convert it to the necessary control signals for the video game 40.

The programming of the mapping computer 36 will depend upon the type of user interface implemented by the video game 40. Since most video games require positional control inputs and fire control inputs, the mapping computer 36 will generally be programmed to map the digital data on bus 34 to the appropriate position control and fire control data words on bus 44 to control the video game 40. Of course for other types of video games which require other types of user input, the mapping computer 36 would be programmed to supply the appropriate type of user input required by the video game 40.

Figure 3 shows a process of collecting electrical signals from a living body and analyzing these signals for certain characteristics and for generating output manifestations in real time based upon these characteristics. The process consists essentially of an input step wherein biologically related electrical signals such as EEG, EMG, EOG, or EKG signals are sensed via electrical transducers in a known manner. This step is symbolized in block 46. Step 46 also symbolizes the process of filtering or otherwise processing the analog signals collected from the body to derive the desired electrical signals where those electrical signals may be obscured by noise or by other signals generated in the body which are not of interest.

Block 48 represents the steps in the process of converting the analog signals derived in step 46 to digital data. Block 48 can represent a straight analog-to-digital conversion of intensity values to corresponding digital values or other types of processing. For example, the frequency of the electrical signals may be converted to an analog signal and the amplitude of that analog signal then converted to a corresponding digital value. Likewise, the changes in frequency or changes in intensity of the analog signals of interest may be derived and then converted to analog signals which have amplitudes corresponding to the change in frequency or change in intensity. The amplitude of these signals may then be converted to corresponding digital data. The particular process that block 48 represents depends on the application and the characteristics of interest of the electrical signals sensed in step 46.

Block 50 represents the process of analyzing the digital data generated in step 48 for one or more characteristics. In other words, block 50 represents the process of determining from the digital data representation of the electrical signals generated by the body certain characteristics of those electrical signals generated by the body. For example, step 50 may represent the averaging of the digital data words over a particular interval representing intensity, changing intensity, frequency or change in frequency. Other forms of processing may also be used such as digital filtering, convolution, correlation, or other well known digital signal processing techniques.

Block 52 represents the process of mapping the characteristic derived in step 50 to digital data which controls a particular parameter or parameters of the output manifestation. In the preferred embodiment where the output device is a music synthesizer, block 52 represents the process of mapping the intensity of the incoming signals to the frequency of musical notes that are generated. For other embodiments where different parameters of the synthesizer are to be controlled, block 52 represents the particular mapping which is desired. For example, the frequency of the incoming analog signal may be mapped to the harmonic generation by the music synthesizer or to the program number. Or, changes in intensity of the incoming signal may be mapped to a corresponding attack rate or decay rate of the musical notes generated. Any one of a number of different characteristics about the incoming electrical signal may be mapped to one or more parameters of the output manifestation which may be controlled.

Block 54 represents the process of generating output control signals or output digital data to implement the mapping performed by the process step symbolized by block 52.

Block 56 represents the process of generating musical notes or other output manifestations in real time based on the digital data or control signals generated by the process steps of block 54.

Although the process depicted in Figure 3 is very general, the teachings of the invention encompass a wide variety of embodiments. There are many options as to the types of electrical signals which may be sensed from the living body, the manner in which those signals are converted to digital data and the manner in which that digital data is analyzed. Further, there are many options as to the form of mapping which may be implemented by the steps symbolized by block 52 and as to the type of output data generated from the mapping process. Also, the type of output device which is used is very application dependent. The specifics of any one of the steps mentioned above will therefore depend upon the particular application in which the teachings of the invention are to be employed. There follows a discussion of a specific embodiment which illustrates the manner in which the teachings of the invention may be employed in a particular application.

Referring to Figure 4, there is shown a block diagram of a specific system which employs the teachings of the invention to map the intensity of incoming biologically generated electrical signals to musical notes which have a corresponding frequency. The circuitry within block 60 represents typical circuitry for one channel of sensing apparatus to sense the electrical signals generated in the human body. Multiple channels may be used. Fundamentally, each channel sensing circuit consists of various gain and filtering stages to process the analog signal to remove noise and unwanted signal components prior to analog-to-digital conversion.

Three transducers 12, 14 and 16 are used for each channel. Transducers 12 and 16 are coupled to the positive and negative inputs of a differential amplifier 62 which has its signal ground connected to a third transducer 14.

The transducers are electrical contacts which are applied to the skin of the subject in a position to sense the desired signal. For example, if EKG signals are to be detected, the transducers will be taped to the skin of the chest of the subject in a known manner to detect the electrical signals generated by the heart muscles.

The differential amplifier 62 is a quiet amplifier with low internal noise generation and a high common mode rejection.

The output of the differential amplifier 62 is coupled to the input of a high pass filter 64. The purpose of the high pass filter is to eliminate the D.C. offset which is present on the output signal line 66 of the differential amplifier 62. The high pass filter has a roll off frequency of 1 Hz to accomplish this function.

The output of the high pass filter 64, in some embodiments, is coupled to the positive input of a single-ended amplifier 68. This amplifier is used as a variable gain stage. The negative input of the amplifier is coupled to the wiper arm of a potentiometer 70 which has one end of the resistive element coupled to ground and the other end coupled to the output of the differential amplifier 68. The purpose of this amplifier is to increase the amplitude level of the electrical signal detected from the body by a user selectable amount. The output of the variable gain stage 68 is coupled to the input of a low pass filter 72.

Low pass filter 72 has a 1 kHz roll off point such that only signals having frequencies less than 1 kHz can pass through this filter without substantial attenuation. Collectively, the filters 64 and 72 represent a band pass filter which passes frequencies higher than 1 Hz and lower than 1 kHz. These roll off frequencies on either end of the passband may be adjusted for the frequency of interest. For example, if alpha waves in an EEG are to be detected, the filter roll off points may be adjusted to pass only the band of frequencies around 10 Hz in which the alpha waves are found.

A multiplexer 74 is needed if more than one channel is present. The multiplexer has numerous inputs, each of which is connected to an output of one of the channels and, specifically, to the low pass filter 72 output in one of the channels. Multiplexer 74 also has a plurality of select inputs which are coupled to a select bus 76. This bus is coupled to the output of an address latch 78 which has its data inputs coupled to the address bus 80 of a signal-processing chip 82. In preferred embodiments, a 320C10 is used. The load control input of the address latch 78 is coupled to the ADDRESS VALID control signal on line 84 from the signal-processing chip. The signal-processing chip therefore controls switching by the multiplexer 74 by writing the address of the channel whose input signal is to be analyzed on the address bus 80 and latching it into the address latch 78 by activating the ADDRESS VALID signal on line 84. This causes a specific bit pattern on the bus 76 which causes the multiplexer 74 to select the input signal line from the desired channel for coupling to an output signal line 88.

The selected analog signal on line 88 is coupled to the input of an analog-to-digital converter 90. The A/ D converter receives a clock signal from the signal processing chip 82 via a clock divider 91 and a line 93. Each upward transition of the clock signal on line 93 turns on the analog-to-digital converter 90 to start the conversion process for the analog signal at the input of the A/D converter. When the analog-to-digital converter 90 has completed its conversion process, it activates the DONE signal on line 94. This signal is latched into a latch 96 acting as a status flag. This latch has its output coupled to the signal-processing chip 82 by the signal line 98. Signal line 98 can be coupled to the interrupt input of the signal-processing chip 82 in some embodiments or it may represent the process of polling the flag latch 96 from time to time to determine whether the conversion is done.

When the signal-processing chip senses the DONE signal, it activates a READ signal on line 92. This signal causes the A/D converter to place the conversion data on the data line 104. The clock divider 91 receives a clock signal from the signal-processing chip 82 and divides it by a factor of 250. The resultant slower clock signal is output on line 93 to the "start conversion" input of the analog-to-digital converter 90.

The signal-processing chip 82 controls the multiplexer 74 and the analog-to-digital converter 90 in such a way as to sample the input signals coming from each channel at a rate which is set by the Nyquist criteria for the expected frequency of the signal on that channel. The Nyquist criteria requires that for effective sampling of a signal of frequency F with no aliasing, the sampling frequency must be at least 2F. In the application shown in Figure 4, the frequencies of interest are in the frequency band from 1 Hz to 1 kHz. Thus, the signal-processing chip 82 controls the sampling of the signal from channel 1 such that the effective sampling rate is 2 kHz.

The output digital data from the analog-to-digital converter 90 is temporarily latched in a buffer 100. The buffer 100 stores the digital data until such time as the signal-processing chip 82 does an I/O transaction to read the contents of the buffer via data bus 102. The READ signal on control line 92 from the signal-processing chip is also coupled to the load control input of the buffer 100. Each time the READ signal is activated, the analog-to-digital converter 90 starts a new conversion and the buffer 100 is loaded with the data from the previous conversion then present on the data output bus 104 of the analog-to-digital converter 90.

The signal-processing chip gathers digital data from each channel in the manner described above. This data is then analyzed and mapped to digital data having MIDI word format for transmission to the output device via a UART 106. The UART has its parallel data input coupled to the data bus 102 of the signal-processing chip 82. The signal-processing chip does I/O transactions with the UART 106 to write parallel data therein. The UART then converts this parallel data to serial format MIDI words and transmits the resultant MIDI word stream on a serial format signal line 108 to the synthesizer.

The signal-processing chip 82 performs its data gathering and mapping function in accordance with the flow chart shown on Figure 5. Referring to Figure 5, there is shown a flow diagram of the preferred embodiment of the invention for an application where the intensity of the electrical signals generated in the body are mapped to the frequency of the notes generated by the music synthesizer. The first step in this process is to reduce the effective sample rate as symbolized by block 110 since many more samples have been taken by the sampling circuitry than are necessary to represent the narrow bandwidth of the biopotential. The lower effective sample rate allows more time for calculation between samples. That is, because the music synthesizer can accept MIDI words at a far slower rate than digital data may be acquired from the body, it is important to slow the effective sampling rate down to a level at which MIDI words can be generated and accepted by the music synthesizer in order to generate musical notes at an acceptable rate. In the preferred embodiment, this is done by the process steps symbolized by block 110. This block symbolizes the process of accepting every sixth sample which is input from the analog-to-digital converter 90. This gives an effective sample rate of 2 kHz from the actual sample rate of 12 kHz at which the multiplexer 74 and the analog-to-digital converter 90 are being driven.

In order to establish an initial collection of data samples from which the mapping process may start, a step 112 is performed. This step represents the process of loading the first 8 samples into a memory array for purposes of averaging. The average intensity over a group of samples is used for the mapping function rather than individual sample values to eliminate the effects of noise in the system and to get smoother transitions in the flow of musical notes which are generated.

The next step is to average the sample values in the array as symbolized by block 114. On the first time through the program loop, there will be only 8 samples in the array, and the average will be over only these 8 samples. However, the array has the capacity to store 32 samples, and in subsequent loops through the step 114, the average will be taken over 32 samples because new samples will have arrived during the time the loop is being traversed.

After the average of the values in the array is computed, a test 116 is performed to determine whether the average is greater than a predetermined threshold. The purpose of this test is to eliminate noise from the mapping process such that noise samples do not create musical notes. If the average is less than the given threshold, a step 118 is performed where the signal-processing chip waits one sample time and then branches back to step 114 via path 120.

If the result of the test of step 116 is that the average is greater than the threshold value, then step 121 is performed. Step 121 represents the process of performing an I/O transaction to the UART 106 in Figure 4 to send a MIDI word which is designated "note on". This tells the output music synthesizer that the signal-processing chip 82 desires to turn on a note. However, the particular note which is to be turned on must also be sent in another MIDI word before the synthesizer can turn on a note. To communicate the particular note that the signal-processing chip wishes to turn one, step 122 is performed. Step 122 represents the process of waiting four sample times (at the effective sample rate) and then sending the note number MIDI word to control which note is played by the music synthesizer.

The mapping process of mapping biopotential intensity (amplitude) to note number is carried out in step 122. This is done by taking the computed average and using that as the note number. This 8 bits a data is put on data lines D8 through D15 of the signal-processing chip data bus coupled to the UART 106. In other embodiments a look up table could be consulted or some mathematical algorithm could be performed.

The third MIDI word in a "note on" MIDI sequence is the "note on velocity"; in other words, to play a musical note, the music synthesizer must receive three MIDI words, one being the "note on" command, one being the "note number" command word to identify the frequency of the note desired, and the third being the "note on velocity" which determines part of the feel or impression of the note. Step 124 represents the process of sending the "note on velocity" after waiting four sample times. In the algorithm shown in Figure 5, the "note on velocity" is fixed at a value half way between the minimum and maximum possible MIDI note on velocity values. In other algorithms, the "note on velocity" word can be generated by mapping from the data on another channel such as an EEG or EKG channel.

After the "note on" MIDI sequence is sent, the note will be allowed to sound for 8 sample times. After 8 sample times, the "note off" MIDI sequence is sent. The first step in this sequence is symbolized by block 126. This step represents an I/O transaction to write the "note off" command word into the UART.

Block 128 represents the process of sending the "note number" MIDI word to the output device to tell it which note to turn off.

The final step in the "note off" sequence is symbolized by block 130 which represents the steps of waiting four sample times and sending the "note off velocity" MIDI word. This word establishes part of the feel or impression created by the note.

It is useful to have musical manifestation of EEG output symbolizing brain waves. The apparatus of Figure 4 can be used to detect the alpha wave component (or other aspects) of brain waves by adjusting the low frequency roll off of the high pass filter 64 to be approximately 7 Hz and adjusting the high frequency roll off of the low pass filter 72 to be approximately 30 Hz. Although it is not mandatory, it is preferred to move the variable gain stage 68 to the output side of the low pass filter 72. The only other changes necessary are to change the programming of the signal-processing chip 82 to implement the method shown in Figure 6.

The first step in the EEG mapping process is the MIDI set up step. This step is common to all the mapping programs disclosed herein but has not been shown in all the flow charts for the sake of brevity. This MIDI set up step represents the process of sending the MIDI program code to the synthesizer to establish a set of MIDI parameters which control the characteristics of the sound. Each set of such parameters has a program number. This effectively establishes the synthesizer as having a certain characteristic sound.

To perform mapping of the alpha wave component of a brain wave, it is first necessary to find the amplitude of the alpha wave component. Alpha waves have an average characteristic frequency of 10 Hz. Since the input signal, after filtering, has frequency components from 7 Hz to 30 Hz, the 10 Hz component must be separated from all the other frequencies in some manner. A fast fourier transform, sometimes called an FFT, is well known in the art, and it will not be described in detail herein. An FFT is performed to separate the 10 Hz component from the rest of the signal. To perform on FFT on a digital computer, a discrete sample set is required. Step 142 represents the process of collecting the number of samples required to perform the FFt. In the preferred embodiment, 8 samples are used for the FFt, although in other embodiments larger numbers of samples may be used.

In the preferred embodiment, the effective sampling rate for EEG mapping is 150 Hz. Since the alpha wave component is only 10 Hz, any sampling rate of 20 Hz or greater will suffice for purposes of practicing the invention.

The next step in the process is symbolized by block 144 which represents the steps of performing the FFT calculation. The FFT determines the amplitude coefficients of the FFt fourier series for a series of harmonics which, when mixed together at the amplitude specified by their coefficients, will combine to create the original signal. In other words, the fourier transform is a transformation from the time domain to the frequency domain. It yields the frequency components in the frequency domain of any signal in the time domain which is subjected to the fourier transform mathematical operation. The purpose of performing the FFt in the method of Figure 6 is to determine the amplitude of the 10 Hz component in the input signal represented by the samples collected in step 142. Step 146 represents the examination of the results of the FFt of step 144 to determine the 10 Hz coefficient value. This coefficient represents the amplitude of the alpha wave component.

Step 148 represents the process of comparing the 10 Hz coefficient value determined in step 146 to a fixed threshold. This is done to prevent alpha-wave components of insufficient magnitude from causing changes in the mapping.

If the 10 Hz component is less than the threshold value, then step 150 is performed. Step 150 represents the process of waiting for a new batch of samples to be collected for purposes of performing a new FFT transform.

If the 10 Hz component is greater than the threshold, then a step 152 is performed. This step represents the process of sending a program change sequence of MIDI code words to the output device to cause the "voice" or sound of the music synthesizer to change. As is well known in the art, music synthesizers and other MIDI machines have prearranged programs or sets of MIDI parameters which cause the machine to have a characteristic sound, such as breaking glass, horns, drums, cymbals, or other known or as yet unknown sounds. Each of these sets of parameter values are identified by a program number. In the method shown in Figure 6, the subject's thought pattern can change the program when the alpha-wave component has a sufficient magnitude. When this occurs, step 152 is performed to send a sequence of MIDI words which causes the program number to be changed. The sequence is only two words long, the first MIDI word being the "change program" command and the MIDI "channel" on which the program is to be changed, and the second word being the program number to which the MIDI machine is to be programmed.

As is well known in the art of generating computer music, the MIDI interface has sixteen channels. Thus, complex music can be performed by sending streams of MIDI "note on" and "note off" sequences, control parameters and MIDI program numbers on each of the sixteen channels. The MIDI machine time-division multiplexes itself among these channels and plays all the notes in accordance with the program and other control information received on a particular channel. Since this appears to happen simultaneously to the user, very complex musical pieces can be generated in this manner.

Of course, those skilled in the art will appreciate that other components of the brain wave may be sensed and mapped to different numbers such that multiple program changes may result from changing thought patterns.

Referring to Figure 7, there is shown a flow diagram of the process implemented by the signal-processing chip 82 in mapping EKG signals into MIDI code which is suitable to control a drum machine. Those skilled in the art will appreciate that a drum machine is not the only output device which could be controlled by the MIDI code generated by the EKG mapping routine of Figure 7 since the mapping function may be adjusted for the type of machine and parameter to be controlled.

The EKG mapping routine starts out with step 160, which is a MIDI set-up step to send the program number to the synthesizer or other MIDI machine to establish its sound quality. Other control parameters are also sent in MIDI code to properly set up the machine.

The fundamental frequency of interest in EKG mapping is in the frequency range of 0.1 Hz to 4 Hz. The intensity of the various components of the EKG signal is determined by the steps symbolized by block 162. This process can be accomplished in any known way, such as by forming the FFT as was done in the EEG mapping of Figure 6, or by digital or analog filtering out of all but the desired component or components and measurement of the intensity. Any known signal processing technique to determine the intensity of the desired frequency components will suffice for the EKG mapping routine, and the same way be said for the EEG mapping routine of Figure 6, except that in that case the amplitude of the 10 Hz alpha-wave component of the brain wave is sought.

Next, the intensity of the desired frequency component of the EKG signal is compared to a fixed threshold, as symbolized by test block 164. If the amplitude is less than the threshold, then step 166 is performed which is simply a process of waiting for another batch of samples to arrive to perform another FFT calculation. If digital or analog filtering is being used to separate out the desired frequency component, then step 166 is skipped, and control is transferred from step 164 directly back to step 162, and step 166 is not performed.

If the amplitude of the desired frequency component is greater than the threshold, a step 168 is performed to send a "note on" sequence to, e.g., a MIDI drum machine. Those skilled in the art will appreciate that other types of MIDI machines may be substituted, or that the mapping need not be from the desired frequency component intensity to a note number. For example, the mapping may be from the desired frequency component intensity to any other MIDI parameter such as program number, control parameter, and/or system exclusive messages, and so no.

After step 168 is performed, a step 170 is performed to send the "note off" sequence to the drum machine after a suitable delay. Those skilled in the art will appreciate that the delay may be any suitable delay set by the user.

Referring to Figure 8, there is shown a flow diagram for a process to modify MIDI code by evaluating the body's reaction to musical notes where these notes were generated in response to biopotentials sensed in the body. Fundamentally, the process shown in Figure 8 evaluates biopotentials at time zero and generates musical notes therefrom. After the musical notes are generated, the body reacts to those notes, and biopotentials in the body change. These body potentials are reevaluated and new MIDI code is generated in response to changes in the biopotentials. This process is implemented starting with step 172, which senses the biopotentials in the manner described above using the apparatus described in Figure 4, or any other apparatus which is capable of sensing biopotentials in the frequency range of interest.

Step 174 represents the process of evaluating the biopotentials for their characteristics and mapping these characteristics to one or more of the various MIDI parameters. After this mapping has occurred, the appropriate MIDI code words are generated and transmitted to the output device.

Step 176 represents the process performed by the output device in generating musical notes in response to the MIDI code generated in step 174. After these musical notes have been generated, the body will react to them. When the body reacts, the brain wave potentials change, and possible the heart rate or EKG potentials will change. Further, the muscle (EMG) may also change if the body moves in response to the notes. Step 178 represents the process of reevaluating the characteristics of these biopotentials to evaluate the changes therein in response to the musical notes generated in step 176. Step 178 also represents the process of evaluating the new characteristics of the biopotentials so sensed and mapping those changes to changes in the MIDI code generated in step 174.

Step 180 represents the process of generating new MIDI code in response to the mapping performed in step 178. Processing then returns to step 172.

Referring to Figure 9, there is shown a flow diagram for a process of modifying incoming MIDI code from another MIDI machine in response to certain characteristics of biopotentials generated in a living body. There are many types of MIDI machines currently available, and moe MIDI machines are appearing constantly. Many of these machines have MIDI code outputs such that musical data generated in the machine may be transmitted in MIDI word format to other machines. Step 182 represents the process of receiving such MIDI code from another MIDI machine. Step 184 represents the process of sensing biopotentials from a living body in real time. Any type of biopotential may be sensed, as described herein or in a fashion other than as described herein.

Step 186 represents the process of evaluating the characteristics of the biopotential so sensed. This evaluation is done in real time. Once the characteristics of the biopotentials are determined, these characteristics are mapped to corresponding modifications to the MIDI parameters of the incoming MIDI code. For example, if the sensed biopotential is an EKG signal, a rising heart rate could be mapped into higher note numbers or changed rhythms on a rhythm machine. Step 188 represents the process of modifying the incoming MIDI code in response to the biopotential characteristics determined in step 186. New MIDI output code is then generated and output.

Referring to Figure 10, there is shown a system according to the teachings of the invention to sense four different kinds of biopotential signals and to receive MIDI code from another machine and generate new MIDI code in multiple channels.

The system maps characteristics of the biopotentials into MIDI code on multiple channels for controlling various parameters of an output device, such as a music synthesizer. The system is comprised of four different front-end channels to sense biopotentials through electrodes placed on the body of a subject. The four channels are an EMG channel, 200, an EEG channel, 202, and EKG channel, 204, and an EOG channel, 206 (to sense eye position). Each of these channels is similar to the channel 1 circuitry shown in Figure 4 at 60. However, each channel may have its own frequency passband, to eliminate any analog components of the biopotential frequencies other than the frequencies of interest.

The system also includes an interface 208 for receiving MIDI code from another MIDI machine. This interface receives and stores incoming MIDI code words in, for example, a FIFO buffer in a UART. When a MIDI code word is in storage, the interface 208 raises an interrupt flag on line 210 to cause the signal-processing chip 82 to read the newly arrived MIDI words via a data bus 212. This I/O transaction would be performed by writing the address of the interface 208 on an address bus 214 coupled to a decoder 216. This decoder would then activate the ENABLE-2 signal on line 218 to cause the internal buffer on the interface 208 to place its MIDI word data on the data bus 212. The signal-processing chip 82 then performs a read I/O transaction to obtain the incoming MIDI code words. The signal-processing chip 82 may also periodically poll the interface 208 instead of using an interrupt structure in other embodiments. In the preferred embodiment, the signal-processing chip 82 time-division multiplexes its services among the four biopotential channels and the interface 208. That is, the signal-processing chip 82 sequentially generate the addresses of each of the biopotential channels and the interface 208 on the address bus 214. The decoder 216 then sequentially generates appropriate select control signals on a bus 220 coupled to the select control input of a multiplexer 74 to cause it to sequentially couple each of the four outputs from the biopotential channels in turn to the output signal line 88 through the multiplexer 74.

An analog-to-digital converter 230 receives the input analog data and converts it to digital data which is written via a bus 232 into a buffer 100. As each channel is selected by the signal-processing chip 82 via the multiplexer 74, the digital data corresponding to the sample from that channel is stored in the buffer 100. The signal-processing chip then does an I/O transaction to read the data in the buffer 100 by writing the address of the buffer on the address bus 214, thereby causing the decoder 216 to activate an ENABLE-1 signal on a line 234.

This causes the buffer 100 to place its data on the data bus 212, and the signal-processing chip then reads it via an I/O read transaction. The signal-processing chip 82 then stores this data in an internal register or scratchpad RAM, or in external RAM 236 for processing.

The mapping function the signal-processing chip 82 performs is highly application-dependent. Suffice it to say that the teachings of the invention contemplate that any mapping performed in real time between a characteristic of a biopotential sensed in real time to a parameter which may be controlled via a MIDI word of a parameter which may be controlled in some other output device will be within the scope of the invention. The invention is not limited to the MIDI interface. Any output devices which have parameters which may be controlled by the user will be within the scope of the invention providing there is no prior art which restricts the scope of the invention to something less. For example, it a machine is developed in the future which can alter the moods of a subject by causing electrical stimulus of the brain or otherwise altering the brain chemistry in some fashion, it is within the scope of the invention to map the biopotential characteristics to parameters of such a machine which may be controlled to alter the mood of the subject by muscle movements or thought patterns.

Signal-processing chip 82 has a service routine for each time slot. The type of mapping routine performed during each time slot depends on the particular type of channel data which is being processed during that time slot. For the EMG channel 200, the mapping routine would be comprised of the steps shown in Figure 5. When EEG channel data is being processed, the mapping routine would be similar to the process shown in Figure 6. When EKG data is being processed, the mapping routine would be similar to the routine shown in Figure 7. When dipole data from the eye from the EOG channel 206 is being processed, the mapping routine performed by the signal-processing chip 82 would be similar to the mapping routine shown in Figure 5. When the signal-processing chip 82 is processing incoming MIDI code from another machine, the mapping routine would be similar to Figure 9.

The MIDI interface code contemplates the use of up to sixteen channels. Thus, sixteen separate MIDI word data streams may be sent to the same device in separate time slots. Thus, the output device may respond virtually simultaneously to up to sixteen different MIDI command word streams and appear to be simultaneously responding to all of them. In reality, the output device is separately responding in sixteen different time slots to sixteen different command word streams. However, since the switching is so rapid, the human ear and brain would tend to integrate the distinct sounds into one composite aural work. To accomplish this time-division multiplexing on the outputs, the signal-processing chip 82 would output the MIDI code generated during each time slot service routine to the output device on the corresponding MIDI channel. This would be done by writing the data for each time slot, i.e., channel to the appropriate UART. Each UART can handle up to 16 MIDI channels. the system can control as many output devices as there are UARTs. These UARTs are shown symbolically at 240. The appropriate output device can be driven by writing the address of the appropriate UART on the address bus. This causes the decoder 216 to activate the appropriate enable signal on the ENABLE-2 bus 242 to cause the UART for the corresponding output device to become enabled. The signal-processing chip 82 would then perform an I/O write transaction to the appropriate UART via the data bus 212. All the other UARTs in the collection of UARTs shown at 240 would have their inputs coupled to the data bus 212 in a high-impedance mode such that the incoming data would be stored only by the UART that is enabled. That UART would then convert the parallel format data from the data bus 212 into a serial stream of MIDI code words which would be output to a music synthesizer or other output device 244 via a serial data link 246.

## Claims

1. An apparatus for generating data for controlling the generation of musical notes comprising:
means for sensing biopotential signals in real time;
means for receiving said biopotentials and mapping them in real time into MIDI code.

2. An apparatus as defined in claim 1 further comprising any known machine capable of receiving and utilizing MIDI code to control the operations of said machine coupled to receive said MIDI code for generation of musical notes therefrom.

3. An apparatus for real time generation of digital data to control the generation of musical notes mapped from electrical signals generated in a living body comprising:
at least one output device for converting incoming digital data for the control of musical notes into musical notes;
means for sensing in real time at least one electrical signal generated in a living body and converting said electrical signal in real time into a first stream of digital data samples representing said electrical signal;
mapping means for receiving said first stream of digital data samples and for extracting in real time from said first stream of digital data samples at least one characteristic of said electrical signal generated in said living body and for mapping in real time each said characteristic and the changes therein into a second stream of digital data capable of controlling the generation of musical notes and for transmitting same to said output device.

4. The apparatus of claim 3 wherein said means for sensing includes a differential amplifier having an output and having an input coupled to sense and to amplify said biological signal via two sensing electrodes and a ground electrode coupled to said inputs, said electrodes for being placed in contract with a body, said differential amplifier having sufficient common mode rejection to substantially eliminate common mode noise at said input.

5. The apparatus of claim 4 further comprising a bandpass filter means coupled to the output of said differential amplifier for passing to said mapping means without substantial attenuation only frequencies within the passband of said bandpass filter means.

6. The apparatus of claim 5 wherein said sensing means further comprises a plurality of data channels each of which includes a differential amplifier and a bandpass filter, at least some of said data channels having different passbands for said bandpass filters, each said channel for sensing a different biopotential signal generated in a living body and each said channel having a signal output.

7. The apparatus of claim 6 further comprising a multiplexer having a plurality of inputs each of which is coupled to said signal output of one of said channels, and having a signal output coupled to supply said electrical signal to said mapping means, and said multiplexer having a control input for controlling which input is coupled to said signal output, said control input coupled to said mapping means to receive select signals therefrom, and wherein said mapping means is also for generating signals to control which said input said multiplexer couples to said output.

8. An apparatus for converting biological electrical signals to musical notes comprising:
input means for sensing and amplifying electrical signals generated by a living body and for filtering out unwanted components of said electrical signals and for converting the remaining components to a first stream of digital data;
processing means for receiving said first stream of digital data and extracting from it one or more characteristics of said electrical signals and mapping in real time each said characteristic and its changes to a MIDI parameter and corresponding changes in said parameter, and for generating a stream of MIDI words for each said characteristic; and

at least one MIDI machine for receiving said stream of MIDI words and for converting said MIDI words to musical notes or other musical data.

9. An apparatus for converting electrical signals generated in a body into video data in real time comprising;
input means for sensing a biological electrical signal and for converting it in real time to a first digital data stream;
a video output device for converting incoming digital data containing user input information into video display information; and
mapping means for receiving said first digital data stream and for extracting a characteristic of said biological electrical signal embodied in said first digital data stream and for mapping said characteristic into a second digital data stream capable of supplying said user input information and for transmitting same to said output device.

10. An apparatus for modifying MIDI code generated by a machine in accordance with biopotentials generated by a living body comprising:
means for receiving MIDI code from another machine;
mapping means for sensing electrical signals generated by a living body;
mapping means for determining a characteristic of said electrical signal and mapping the characteristic to a change to be made in said received MIDI code; and
means coupled to receive said MIDI code from said other machine and data from said mapping means regarding the change to be made in said MIDI code and for making the change required by said data from said mapping means and for outputting MIDI code modified in accordance with signals from said mapping means.

11. An apparatus for mapping one or more frequency components of brain waves to MIDI code comprising:
means for sensing brain waves and for filtering out frequencies outside a passband including the characteristic frequency .of the desired frequency component and for outputting the filtered output signal;
means for sampling said filtered output signal at a rate which is sufficiently high to prevent aliasing;
means for performing a fourier transform operation on a plurality of said samples;
means for determining the amplitude of the desired frequency component coefficient resulting from said fourier transform operation at the characteristic frequency of said desired frequency component;
means for mapping the intensity of the desired frequency component into MIDI code.

12. The apparatus of claim 13 wherein said means for mapping includes means for mapping changes in intensity of the alpha wave component of brain waves to changes in the program number of the MIDI code.

13. An apparatus for generating MIDI code from electrical signals generated in a human body comprising:
means for sensing electrical signals generated in a muscle or group of muscles in a living body and for generating a first output signal;
means for sensing brain waves generated in a living body and for filtering out unwanted frequency components and for generating a second output signal;
means for sensing electrical signals generated in a living body to drive a heart muscle and for filtering out unwanted frequency components and for generating a third output signal;
means for sampling said first, second and third output signals at sample rates which are high enough to prevent aliasing and for generating digital data representative of each of said first, second and third output signals; and
processing means having an input for receiving said digital data and having an output, said processing means for extracting a characteristic of each of said first, second and third output signals from said digital data and for mapping each said characteristic to a different channel of MIDI code and for outputting each said channel of MIDI code at said output.

14. The apparatus of claim 13 wherein each channel of MIDI code is output during a different time interval at said output.

15. A method of generating MIDI code comprising the steps of:
sensing electrical signals generated in a living body; and
mapping said electrical signals to MIDI code.

16. A method of generating digital code for controlling a video game comprising the steps of:
sensing electrical signals generated in a living body; and
mapping said electrical signals to digital code suitable for supplying user input data for a video game.

17. A method of generating MIDI code comprising the steps of:
sensing electrical signals generated in a living body and filtering out unwanted frequency components;
converting said filtered electrical signals to digital data;
extracting at least one characteristic of said electrical signals from said digital data; and
mapping said characteristic of said electrical signals into MIDI code.

18. The method of claim 17 further comprising the step of generating musical notes from said MIDI code.

19. A method of generating MIDI code from brain waves comprising the steps of:

sensing electrical brain waves in real time;

sampling the brain waves at a sample rate which is at least high enough to prevent aliasing and converting the samples to digital data;

performing a fourier transform on the digital data of a plurality of said samples;

determining the magnitude of the fourier series coefficient for the frequency component of interest; and

mapping the magnitude of the fourier series coefficient of the frequency component of interest into MIDI code.

20. The method of claim 19 further comprising the step of filtering out frequency components outside a passband of frequencies wherein the characteristic frequency of desired frequencies is within said passband prior to performing the sampling step, and wherein the step of determining the magnitude of the coefficient of the frequency component of interest comprises determining the magnitude of the fourier series coefficient for the frequency component having the characteristic frequency of alpha waves such as beta, delta and theta waves.

21. The method of claim 19 further comprising the step of filtering out frequency components outside a passband of frequencies wherein the characteristic frequency of EKG waves is within said passband prior to performing the sampling step, and wherein the step of determining the magnitude of the coefficient of the frequency component of interest comprises determining the magnitude of the fourier series coefficient for the frequency component having the characteristic frequency of EKG waves.

22. The method of modifying MIDI code comprising the steps of:

sensing electrical signals generated in a living body;

mapping said electrical signals to MIDI code;

using said MIDI code to generate musical notes;

reevaluating said electrical signals generated in said living body for the effect said musical notes had on said electrical signals; and

modifying said MIDI code by mapping the changes in said electrical signals to corresponding changes in said MIDI code.

23. A method of modifying MIDI code received from another machine capable of generating MIDI code comprising the steps of:

sensing electrical signals generated in a living body;

receiving said MIDI code from another MIDI machine;

evaluating the characteristics of said electrical signals and mapping said characteristics to changes in said MIDI code; and

changing the MIDI code in accordance with the changes determined in said mapping step.